Europäisches Patentamt

European Patent Office

Office européen des brevets

(19)

(11) Numéro de publication: **0 228 965**
**B1**

(12)
# FASCICULE DE BREVET EUROPEEN

(45) Date de publication du fascicule du brevet:
20.06.90

(21) Numéro de dépôt: **86402896.4**

(22) Date de dépôt: **22.12.86**

(51) Int. Cl.⁵: **A61B 17/06, D05B 91/14**

(54) Dévidoir, notamment pour fils de sutures et ligatures chirurgicales.

(30) Priorité: **24.12.85 FR 8519120**

(43) Date de publication de la demande:
**15.07.87 Bulletin 87/29**

(45) Mention de la délivrance du brevet:
**20.06.90 Bulletin 90/25**

(84) Etats contractants désignés:
**AT BE CH DE ES FR GB GR IT LI LU NL SE**

(56) Documents cités:
**FR-A- 2 014 380**
**FR-A- 2 283 974**
**FR-A- 2 308 724**
**FR-A- 2 346 483**
**FR-A- 2 427 415**
**US-A- 3 815 843**

(73) Titulaire: **SOCIETE DES SUTURES CHIRURGICALES
ROBERT ET CARRIERE - LEDERLE, 60, rue de la
Glacière, F-75621 Paris Cédex 13(FR)**

(72) Inventeur: **Yvelin, Robert, 10, rue Celos,
F-27300 Bernay(FR)**

(74) Mandataire: **Bonnetat, Christian et al, CABINET
BONNETAT 23, Rue de Léningrad, F-75008 Paris(FR)**

ACTORUM AG

**Description**

La présente invention a pour objet un dévidoir, notamment pour fils de sutures et ligatures chirurgicales.

On sait que, pour refermer les incisions qu'il pratique, ou pour ligaturer des vaisseaux ou isoler des masses de tissus, un chirurgien utilise des sutures et ligatures, qui permettent le rapprochement des tissus pendant la période critique de la cicatrisation.

Ces fils ont des origines diverses, par exemple animale, végétale, minérale, ou synthétique. Ils peuvent être incolores ou teintés. Leurs calibres peuvent varier, pour les usages les plus courants, entre 50/100 et 100/100 de mm.

Ils sont commercialisés principalement sous deux formes principales, à savoir :
- les fils sur lesquels sont pré-montées des aiguilles, et
- les fils sans aiguilles.

C'est cette dernière forme qui est principalement concernée par la présente invention.

Dans le cas des fils sans aiguilles, la longueur de fil la plus usitée est de 2,50 m. Pour utiliser le fil, le chirurgien a recours à une aiguille montée sur un manche (aiguille amovible, dont il existe une grande diversité), portant le nom de "Reverdin", ou à une aiguille à chas du type "couturier".

L'instrument ainsi constitué s'utilise un peu à la manière d'un passe-lacet.

La réserve de fil est contenue dans un dévidoir dans lequel le chirurgien puise à plusieurs reprises au cours d'une intervention, en prélevant les longueurs qui lui sont nécessaires pour réaliser les points de suture ou les ligatures.

De très nombreuses présentations ont été imaginées pour les dévidoirs de fils chirurgicaux. Les plus anciens consistent en des tubes dans lesquels les fils sont enroulés en hélice, en plaquettes dans lesquelles les fils sont piqués en zigzag, ou en pochettes à l'intérieur desquelles les fils sont lovés en forme de huit.

Ces supports présentent l'inconvénient de donner au fil une déformation temporaire, gênante au moment de l'utilisation : vrillages, "faux plis", etc...

C'est pourquoi la tendance actuelle est de présenter les fils de sutures et de ligatures sur des bobines de diamètre relativement grand par rapport à celui du fil: lorsque ce dernier est dévidé de son support, il est assez peu déformé, et, en tous cas, de manière régulière sur toute sa longueur.

L'ensemble d'un dévidoir de ce type se présente donc comme une bobine cylindrique plate, c'est-à-dire d'un diamètre sensiblement supérieur à l'épaisseur mesurée selon l'axe de rotation, et contenue dans un boîtier pouvant facilement tenir dans la main, et dans lequel elle peut tourner.

En outre, pour être d'un maniement plus commode, les dévidoirs à bobines connus sont souvent munis d'un organe de préhension, tel qu'une poignée ou un anneau pouvant se glisser sur un doigt.

Des dévidoirs correspondant à la description générale ci-dessus sont décrits par exemple dans les documents FR-A-2 014 380, FR-A-2 283 974, FR-A-2 308 724, FR-A-2 346 483, FR-A-2 427 415 et US-A-3 815 843.

Ce type de dévidoir connu, muni d'un organe de préhension, présente toutefois divers inconvénients auxquels la présente invention vise à remédier.

Un premier inconvénient est dû aux habitudes, aux tours de mains différents selon les chirurgiens utilisateurs : les uns préfèrent une simple bobine dans un boîtier plat de forme sensiblement cylindrique, sans autre accessoire et pouvant tenir tout entier dans le creux de la main, tandis que les autres apprécient la présence d'un organe de préhension.

Pour satisfaire les deux tendances, il est donc nécessaire de distribuer deux types de dévidoirs, à savoir l'un avec et l'autre sans organe de préhension.

Un autre inconvénient est dû au fait que, la bobine étant contenue dans un boîtier, ce dernier doit présenter un orifice par lequel le fil pourra être prélevé lors de son utilisation, mais par lequel aussi l'extrémité libre du fil devra être passée lors de la mise en place de la bobine pleine dans son boîtier par le fabricant du dévidoir. Selon les dimensions et la disposition de sortie du fil, il est donc plus ou moins facile de procéder à l'assemblage de la bobine pleine de fil et du boîtier.

Ces inconvénients, ainsi que d'autres qui seront évoqués plus loin, sont écartés grâce au dévidoir conforme à la présente invention ou à des modes de réalisation préférés. A cette fin, selon l'invention, le dévidoir, notamment pour un fil chirurgical de suture ou de ligature, comportant un boîtier de forme sensiblement cylindrique, une bobine contenant ledit fil et logée à l'intérieur dudit boîtier par rapport auquel elle peut tourner, ainsi qu'un organe de préhension solidaire dudit boîtier, est remarquable en ce qu'il comporte des moyens d'articulation dudit organe de préhension par rapport audit boîtier, de façon que ledit organe de préhension puisse prendre soit une position escamotée pour laquelle ledit organe de préhension est rabattu le long dudit boîtier, soit une position déployée pour laquelle ledit organe de préhension fait saillie par rapport audit boîtier.

Ainsi, selon ses habitudes, un chirurgien peut replier ledit organe de préhension pour tenir l'ensemble du dévidoir dans sa main, ou bien déployer cet organe de préhension afin de tenir ledit dévidoir par ce dernier. Il est donc possible de fabriquer un seul type de dévidoir.

De préférence, en position déployée, ledit organe de préhension est au moins approximativement en prolongement du fond dudit boîtier, tandis que, en position escamotée, ledit organe de préhension est replié contre la face extérieure du fond dudit boîtier.

Il est avantageux, notamment afin que les deux positions que peut prendre ledit organe de préhension soient stables et que le passage de l'une à l'autre soit automatique, que lesdits moyens d'articulation soient formés par une charnière à effet de ressort.

Par ailleurs, le dévidoir selon l'invention comporte de préférence des moyens assurant le maintien dudit organe de préhension en position escamotée.

De tels moyens de maintien pourraient être du type à téton encliquetable. Toutefois, dans un mode avantageux de réalisation, lesdits moyens de maintien comportent une empreinte dudit organe de préhension, pratiquée dans ledit boîtier. Ainsi, ledit organe de préhension peut s'ajuster à frottement dans ladite empreinte, de sorte que ledit organe de préhension est fixé par emboîtement dans ladite empreinte.

Ledit organe de préhension peut présenter la forme d'un T, dont la branche verticale est destinée à passer entre deux doigts de l'utilisateur, de sorte que celui-ci peut replier ses doigts autour dudit boîtier.

Afin d'éviter l'inconvénient mentionné ci-dessus concernant la mise en service d'une bobine pleine, lorsque ledit dévidoir comporte un orifice de sortie pour ledit fil prévu dans la paroi cylindrique dudit boîtier, il est avantageux que ledit orifice de sortie soit une fente pratiquée selon la directrice de ladite paroi cylindrique du boîtier et laissant subsister un arceau assurant la continuité avec le reste dudit dévidoir. De préférence, ladite fente est symétrique par rapport au plan de symétrie du dévidoir. Son étendue angulaire peut être voisine de 150°.

Les figures du dessin annexé feront bien comprendre comment l'invention peut être réalisée. Sur ces figures, des références identiques désignent des éléments semblables.

La figure 1 est une vue en perspective agrandie et éclatée d'un dévidoir selon l'invention, la bobine étant représentée démontée dudit boîtier.

La figure 2 est une vue de face, fortement agrandie, d'un mode de réalisation du boîtier du dévidoir conforme à la présente invention, l'organe de préhension étant en position déployée.

La figure 3 est une coupe longitudinale axiale, selon la ligne III-III de la figure 2.

La figure 4 est une coupe transversale selon la ligne IV-IV de la figure 2.

La figure 5 est une vue en coupe axiale correspondant à la figure 3, l'organe de préhension étant en position escamotée.

Sur ces figures, comme il a été dit, le dévidoir est représenté à des échelles très agrandies. En pratique, les dimensions du dévidoir sont déterminées par le fait qu'il doit tenir commodément dans une main pratiquement fermée, tout en étant susceptible de contenir une longueur de 2,50 m de fil, quel qu'en soit le diamètre.

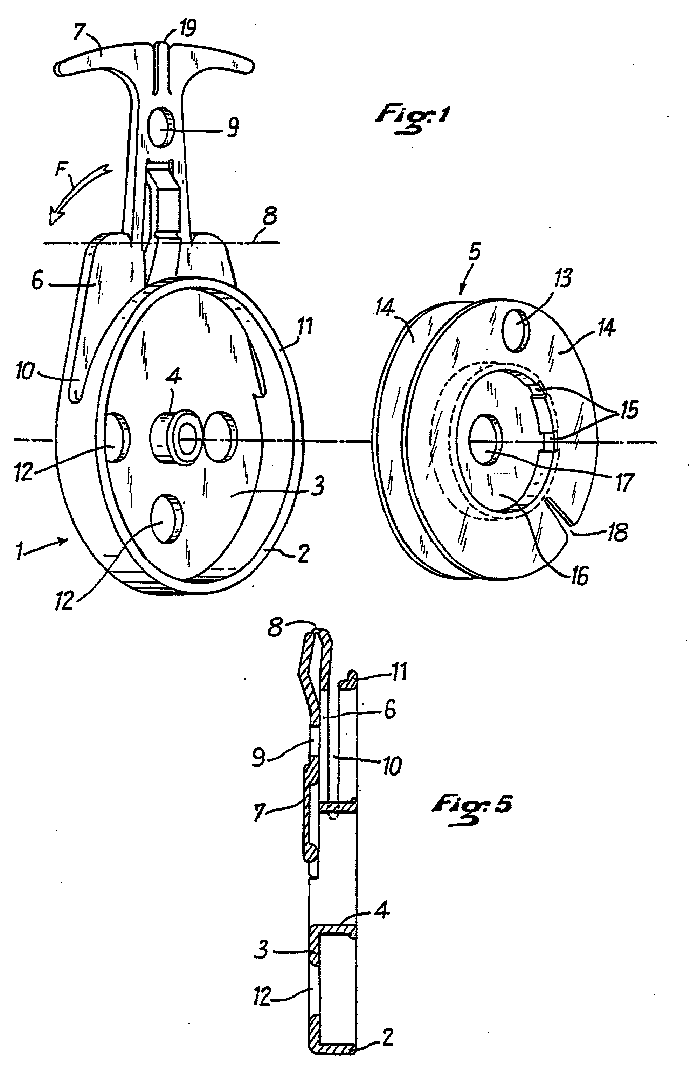

Le boîtier 1 pour le dévidoir selon l'invention, montré par la figure 1, est constitué essentiellement d'une paroi latérale 2, de forme générale cylindrique de révolution, et d'un fond plat 3.

Ce fond 3 comporte, en son centre, un téton axial 4 pouvant servir d'arbre de rotation à la bobine 5, laquelle bobine 5 vient se placer par encliquetage sur ledit téton 4.

Le fond 3 du boîtier 1 comporte de plus un prolongement 6 qui, lui-même, se prolonge par une pièce 7 en forme de T. Cette pièce 7, en forme de T, constitue un organe de préhension pour l'ensemble du dévidoir. En effet, le dévidoir ainsi conformé peut être tenu dans une main, le boîtier proprement dit, c'est-à-dire sa partie cylindrique, se trouvant à l'intérieur de la main et la partie transversale de la pièce 7 en forme de T se trouvant à l'extérieur de ladite main. En d'autres termes, l'ensemble est fermement maintenu par deux doigts de l'utilisateur enserrant la branche verticale du T constitué par la pièce 7, quel que soit le degré d'ouverture de la main.

Par ailleurs, l'absence de moyen de retenue plus contraignant, tel qu'un anneau fermé, permet le dégagement très rapide du dévidoir sans l'intervention de l'autre main.

Le dévidoir, selon l'invention, grâce à son organe de préhension en forme de T, donne toute satisfaction aux utilisateurs souhaitant la présence d'un moyen de préhension.

Pour les utilisateurs préférant les dévidoirs tenant tout entier dans le creux de la main, l'invention prévoit que la pièce 7 en forme de T peut être repliée, ou rabattue, de 180° dans le sens de la flèche F, comme l'une des ailes d'une charnière, autour d'un axe transversal de pivotement 8, de telle sorte que la pièce 7 vienne se plaquer contre la face extérieure du fond 3 bu boîtier 1.

La pièce 7 étant ainsi rabattue, le dévidoir se présente sous forme d'un boîtier plat sensiblement cylindrique duquel dépasse juste le prolongement 6 du fond 3, c'est-à-dire sous forme d'un boîtier de type connu, sans organe de préhension.

Pour assurer le parfait maintien de la pièce 7 rabattue au contact du fond 3, on peut, selon l'invention, prévoir divers moyens connus.

Par exemple, la pièce 7 est munie d'une perforation 9 coopérant avec un téton d'encliquetage (non représenté) solidaire de la face extérieure du fond 3, et placé en regard de la perforation 9 lorsque la pièce 6 est repliée.

On pourrait évidemment inverser la disposition, c'est-à-dire qu'alors un téton solidaire de la pièce 7 viendrait s'encliqueter dans un trou du fond 3.

On préfère cependant la variante avec un trou 9 dans la pièce 7, car ce même trou peut permettre de suspendre le dévidoir à proximité du champ opératoire, lorsque le chirurgien souhaite ne pas tenir en main le dévidoir.

Comme on le verra ci-après, on peut, en variante, prévoir dans le fond 3 une empreinte (non représentée sur la figure 1) de forme conjuguée à celle de la pièce 7, de sorte que celle-ci vienne s'emboîter exactement dans l'épaisseur du fond 3 et y soit maintenue par friction.

Par ailleurs, à une telle fin de maintien de la pièce 7 en position repliée, il est avantageux de munir le pied de la pièce 7 en forme de T d'une charnière à effet de ressort qui relie ladite pièce 7 au prolongement 6 et grâce à laquelle la pièce 7 ne peut occuper que deux positions stables, formant entre elles un angle de 180°. Sur la figure 1, la pièce 7 est représentée dans sa position pour laquelle elle se trouve dans le prolongement du fond 3 ; l'autre position stable (non illustrée sur la figure 1) est celle pour laquelle la pièce 7 est au contact du fond 3.

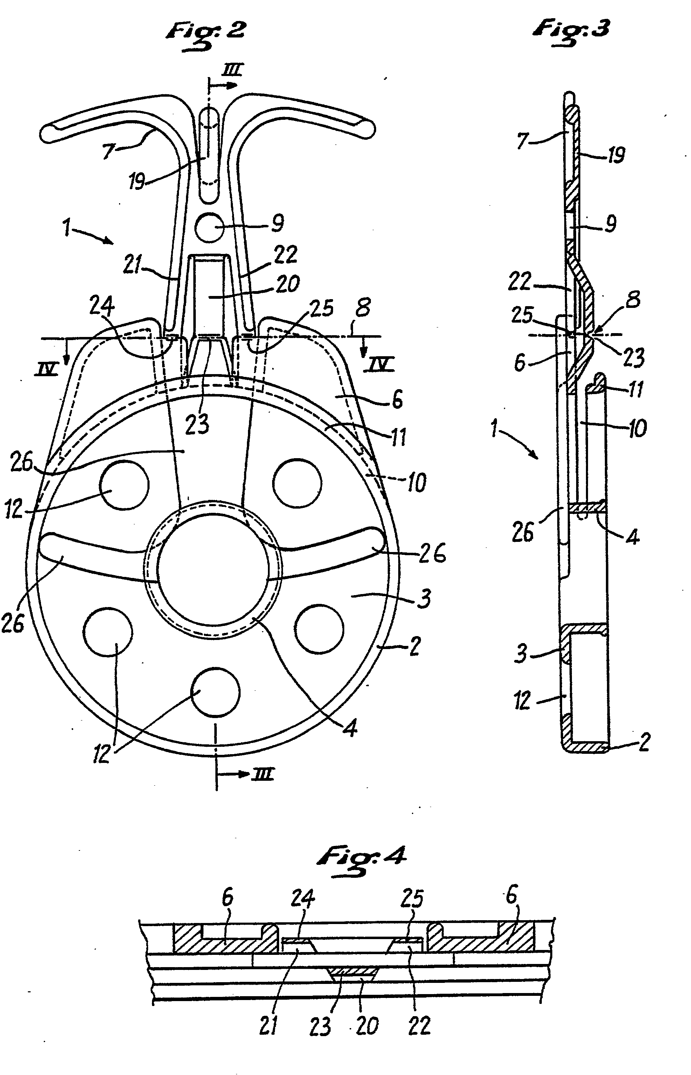

Comme cela est montré sur les figures 2 à 5, une telle charnière à effet de ressort peut se combiner avantageusement avec le maintien par emboîtement de la pièce 7 dans une empreinte de forme conjuguée pratiquée dans le fond 3. Il est en effet possible ainsi d'obtenir, lorsque la pièce 7 est repliée, une surface parfaitement plane, c'est-à-dire sans aspérités, à l'extérieur du fond 3 dans lequel s'insère la pièce 7 comme un élément d'un puzzle.

Selon une autre particularité d'un mode de réalisation préféré de l'invention, l'orifice de sortie du fil (non représenté) provenant de la bobine 5 se présente comme une fente 10 pratiquée selon une directrice de la paroi latérale cylindrique 2, sur un angle au centre de préférence égal à environ 150°, et symétrique par rapport au plan de symétrie de l'ensemble, au voisinage du prolongement 6 supportant la pièce 7.

Les deux bords de la fente 10 ainsi pratiquée sont donc matérialisés d'une part par le fond 3 du boîtier, et d'autre par par un arceau 11 assurant la continuité avec le reste de la paroi latérale 2.

Grâce à la longueur de la fente 10, on peut prélever du fil en le tirant pratiquement dans une direction quelconque, au contraire de ce qui se passe lorsque l'orifice de sortie du fil est constitué d'une simple perforation sensiblement circulaire. Par ailleurs, la longueur de la fente 10 donne une certaine symétrie à l'ensemble du dévidoir, alors qu'une petite perforation se trouve nécessairement au dehors du plan de symétrie, en raison de la non-symétrie par rapport à ce plan, de l'enroulement du fil, quel qu'en soit le sens. La symétrie obtenue selon l'invention permet indifféremment l'utilisation du dévidoir, quelle que soit sa position dans la main (contact de la paume avec le fond 3 ou avec la bobine), et quelle que soit la main tenant le dévidoir (utilisateurs droitiers ou gauchers).

En outre, la grande dimension de la fente 10 facilite la sortie de l'extrémité libre du fil, lors de l'insertion de la bobine pleine dans le boîtier 1.

Le dévidoir selon l'invention peut en outre présenter diverses particularités déjà connues dans le domaine.

Ainsi, le boîtier 1 peut présenter des orifices 12, par exemple dans le fond 3, facilitant la circulation d'un fluide de stérilisation, avant le conditionnement du dévidoir. Des perforations analogues 13 peuvent être également pratiquées dans les joues 14 de la bobine 5, ou encore dans le fond de la gorge de la bobine, comme celles portant les références 15, dans le cas où le centre de la bobine est constitué d'un voile unique 16 percé d'un orifice central 17 permettant l'encliquetage sur le téton 4 du boîtier 1.

L'une au moins des joues 14 de la bobine 5 peut encore présenter au moins une fente ouverte 18, en forme de coin, dans laquelle on peut coincer l'extrémité libre du fil lors du remplissage de la bobine. Après enroulement du fil, cette extrémité libre qui dépasse latéralement est sectionnée à ras, afin de ne pas gêner la rotation de la bobine dans son boîtier. Une autre possibilité d'introduction de l'extrémité du fil est fournie par les lumières 15 pratiquées au fond de la gorge et servant également à la circulation du fluide de stérilisation.

Le diamètre mesuré au fond de la gorge de la bobine dépend du diamètre du fil qu'elle contient. Il est en effet avantageux que le diamètre moyen de l'enroulement soit le plus grand possible, tant pour faciliter le déroulement que pour cintrer le moins possible les fils susceptibles de garder une déformation après déroulement de la bobine.

Pour les fils fins, on choisit donc une bobine à gorge peu profonde, avec un voile 16 de grand diamètre, tandis que, pour les fils les plus gros, la gorge est plus profonde et le diamètre du voile 16 plus petit, l'épaisseur de la bobine, le diamètre extérieur des joues et celui de l'orifice central 17 restant bien entendu les mêmes dans tous les cas, afin que les divers types de bobines puissent être placés dans un seul type de boîtier 1.

Le dévidoir selon l'invention peut également être muni d'un moyen destiné à coincer l'extrémité libre du fil, pour en empêcher le débobinage intempestif. Ainsi, par exemple, une languette souple 19 peut être découpée par exemple dans la pièce 7, ce qui laisse de part et d'autre de ladite languette une fente pouvant recevoir le fil. Une telle languette 19 est préférable à une simple fente qui, lorsqu'on utilise un matériau relativement rigide pour réaliser le dévidoir, ne permettrait pas de maintenir des fils de diamètres très divers. Le moyen de coincement du fil se trouve de préférence au sommet de la pièce 7, de sorte que l'extrémité libre du fil se trouve à l'extérieur de la main, lorsque le dévidoir est utilisé dans la position dépliée illustrée par la figure 1. Les matériaux constitutifs du boîtier et de la bobine sont ceux qui sont déjà connus pour ce type d'application. Ce sont des matériaux plastiques permettant le moulage par injection et présentant des propriétés physiques, mécaniques, physiologiques et économiques requises.

Ainsi, par exemple, le boîtier peut être en une seule pièce de polypropylène, de polyéthylène, de polychlorure de vinyle, etc. tandis que la bobine, qui doit de préférence être transparente pour permettre d'en estimer le contenu, peut être également en polypropylène ou en polyéthylène, ou encore en polystyrène, en polycarbonate, etc.

Sur les figures 2 à 5, on a représenté un mode de réalisation particulier du boîtier 1. On y retrouve les différents éléments 2 à 4, 6 à 12 et 19 décrits ci-dessus en regard de la figure 1.

Dans ce mode de réalisation, l'articulation entre la pièce 7 et le prolongement 6 autour de l'axe 8 est obtenue au moyen de trois languettes 20,21 et 22 présentant des lignes de moindre épaisseur 23,24 et 25, les reliant au prolongement 6.

La languette 20 est centrale et est entourée des languettes 21 et 22, et les lignes 24 et 25 sont alignées, mais décalées par rapport à la ligne 23, parallèle auxdites lignes 24 et 25.

L'ensemble des languettes 20,21 et 22 et des lignes de pliage de moindre épaisseur 23,24 et 25 forme, de façon connue une charnière à effet de ressort.

Ainsi, la pièce de préhension 7 peut prendre l'une ou l'autre des positions stables représentées respectivement par les figures 3 et 5. Sur la figure 3, la pièce 7 se trouve en saillie par rapport au boîtier cylindrique 2 et en prolongement du fond 3 de celui-ci. Sur la figure 5, la pièce 7 est rabattue contre ledit fond 3 et est engagée à friction dans une empreinte 26, pratiquée dans ledit fond. Dans le mode de réalisation représenté, l'empreinte 26 s'étend sur toute l'épaisseur dudit fond 3.

## Revendications

1 - Dévidoir, notamment pour un fil chirurgical de suture ou de ligature, comportant un boîtier (1) de forme sensiblement cylindrique, une bobine (5) contenant ledit fil et logée à l'intérieur dudit boîtier par rapport auquel elle peut tourner, ainsi qu'un organe de préhension (7) solidaire dudit boîtier, caractérisé en ce qu'il comporte des moyens d'articulation (20-25) dudit organe de préhension (7) par rapport audit boîtier (1), de façon que ledit organe de préhension (7) puisse prendre soit une position escamotée pour laquelle ledit organe de préhension est rabattu le long dudit boîtier (1), soit une position déployée pour laquelle ledit organe de préhension fait saillie par rapport audit boîtier.

2 - Dévidoir selon la revendication 1, caractérisé en ce que, en position déployée, ledit organe de préhension (7) est au moins approximativement en prolongement du fond dudit boîtier.

3 - Dévidoir selon l'une quelconque des revendications 1 ou 2, caractérisé en ce que, en position escamotée, ledit organe de préhension (7) est replié contre la face extérieure du fond (3) dudit boîtier.

4 - Dévidoir selon l'une quelconque des revendications 1 à 3, caractérisé en ce que lesdits moyens d'articulation (20-25) sont formés par une charnière à effet de ressort.

5 - Dévidoir selon l'une quelconque des revendications 1 à 4, caractérisé en ce qu'il comporte des moyens (9-26) assurant le maintien dudit organe de préhension (7) en position escamotée.

6 - Dévidoir selon la revendication 5, caractérisé en ce que lesdits moyens de maintien comportent une empreinte (26) dudit organe de préhension (7), pratiquée dans ledit boîtier (1).

7 - Dévidoir selon l'une quelconque des revendications 1 à 6, caractérisé en ce que ledit organe de préhension (7) présente la forme d'un T, dont la branche verticale passe entre deux doigts de l'utilisateur, qui replie ses doigts autour dudit boîtier (1).

8 - Dévidoir selon l'une quelconque des revendications 1 à 7, dans lequel un orifice de sortie pour ledit fil est prévu dans la paroi cylindrique dudit boîtier, caractérisé en ce que ledit orifice de sortie est une fente (10) pratiquée selon la directrice de ladite paroi cylindrique du boîtier, et laissant subsister un arceau (11) assurant la continuité avec le reste dudit dévidoir.

9 - Dévidoir selon la revendication 8, présentant un plan de symétrie, caractérisé en ce que ladite fente (10) est symétrique par rapport au plan de symétrie dudit dévidoir.

10 - Dévidoir selon l'une quelconque des revendications 8 ou 9, caractérisé en ce que l'étendue angulaire de ladite fente (10) est voisine de 150°.

## Patentansprüche

1. Spulapparat, insbesondere für chirurgisches Naht- oder Abbindematerial, bestehend aus einem Gehäuse (1) allgemein zylindrischer Form, einer Spule (5), die den Faden enthält und im Innern des Gehäuses angeordnet ist, demgegenüber sie drehbar ist, und aus einem Greiforgan (7), das am Gehäuse befestigt ist, dadurch gekennzeichnet, daß er Gelenkmittel (20–25) des Greiforgans (7) gegenüber dem Gehäuse (1) aufweist, derart, daß das Greiforgang (7) entweder eine eingefahrene Stellung, bei der das Greiforgan längs des Gehäuses (1) umgeklappt ist, oder eine ausgefahrene Stellung einnehmen kann, bei der das Greiforgan gegenüber dem Gehäuse heraussteht.

2. Spulapparat nach Anspruch 1, dadurch gekennzeichnet, daß in der ausgefahrenen Stellung das Greiforgan (7) sich zumindest annähernd in der Verlängerung des tiefsten Teils des Gehäuses befindet.

3. Spulapparat nach einem der vorhergehenden Ansprüche 1 oder 2, dadurch gekennzeichnet, daß in der eingefahrenen Stellung das Greiforgan (7) gegen die Außenfläche des tiefsten Teils (3) des Gehäuses umgeklappt ist.

4. Spulapparat nach einem der vorhergehenden Ansprüche 1 bis 3, dadurch gekennzeichnet, daß die Gelenkmittel (20–25) aus einem Federkraft-Drehgelenk gebildet sind.

5. Spulapparat nach einem der vorhergehenden Ansprüche 1 bis 4, dadurch gekennzeichnet, daß er Mittel (9-26) aufweist, durch die die Beibehaltung des Greiforgans (7) in der eingefahrenen Stellung gewährleistet wird.

6. Spulapparat nach Anspruch 5, dadurch gekennzeichnet, daß die Mittel der Beibehaltung eine Vertiefung (26) des Greiforgans (7) aufweisen, die in das Gehäuse (1) eingelassen ist.

7. Spulapparat nach einem der vorhergehenden Ansprüche 1 bis 6, dadurch gekennzeichnet, daß das Greiforgan (7) die Form eines T's bietet, dessen vertikaler Zweig zwischen zwei Fingern des Benutzers hindurchläuft, der seine Finger um das Gehäuse (1) herumlegt.

8. Spulapparat nach einem der vorhergehenden Ansprüche 1 bis 7, in dem eine Ausgangsöffnung für den Faden in der zylinderförmigen Wand des Gehäuses vorgesehen ist, dadurch gekennzeichnet, daß die Ausgangsöffnung ein Spalt (10) ist, der längs der Leitlinie der zylinderförmigen Wand des Gehäuses eingelassen ist und einen Bogen (11) bestehen läßt, durch den die Kontinuität mit dem Rest des Spulapparats gewährleistet ist.

9. Spulapparat nach Anspruch 8 mit einer symmetrischen Ebene, dadurch gekennzeichnet, daß der Spalt (10) gegenüber der symmetrischen Ebene des Spulapparats symmetrisch ist.

10. Spulapparat nach einem der vorhergehenden Ansprüche 8 oder 9, dadurch gekennzeichnet, daß das Winkelmaß des Spalts (10) in der Nähe von 150° liegt.

## Claims

1. Reel, in particular for a surgical suture or ligature thread, comprising a casing (1) of substantially cylindrical form, a bobbin (5) containing the said thread and housed inside the said casing relative to which it can turn, as well as a gripping member (7) integral with the said casing, characterized in that it comprises means (20–25) for articulating the said gripping member (7) relative to the said casing (1), in such a way that the said gripping member (7) can assume either a retracted position in which the said gripping member is turned down along the said casing (1), or a deployed position in which the said gripping member projects relative to the said casing.

2. Reel according to claim 1, characterized in that, in the deployed position, the said gripping member (7) is at least approximately an extension of the base of the said casing.

3. Reel according to any one of claims 1 or 2, characterized in that, in the retracted position, the said gripping member (7) is folded back against the outer face of the base (3) of the said casing.

4. Reel according to any one of claims 1 to 3, characterized in that the said articulation means (20–25) are formed by a spring-action hinge.

5. Reel according to any one of claims 1 to 4, characterized in that it comprises means (9–26) ensuring that the said gripping member (7) is retained in the retracted position.

6. Reel according to claim 5, characterized in that the said retention means comprise an imprint (26) of the said gripping member (7), made in the said casing (1).

7. Reel according to any one of claims 1 to 6, characterized in that the said gripping member (7) has the form of a T, whose vertical branch passes between two of the fingers of the user, who tucks his fingers around the said casing (1).

8. Reel according to any one of claims 1 to 7, in which an outlet opening for the said thread is provided in the cylindrical wall of the said casing, characterized in that the said outlet opening is a slot (10) made along the directrix of the said cylindrical wall of the casing, and leaving an arch (11) providing for continuity with the remainder of the said reel.

9. Reel according to claim 8, having a plane of symmetry, characterized in that the said slot (10) is symmetrical relative to the plane of symmetry of the said reel.

10. Reel according to any one of claims 8 or 9, characterized in that the angular range of the said slot (10) is close to 150°.

*Fig.1*

*Fig.5*

**Fig. 2**

**Fig. 3**

**Fig. 4**